# FASCICULE DE BREVET EUROPEEN

(11) **EP 2 697 253 B1**
(45) Date de publication et mention de la délivrance du brevet: **20.09.2017**
(21) Numéro de dépôt: 12722415.2
(22) Date de dépôt: 12.04.2012
(51) Int. Cl.: C07K 14/47, A61K 38/17, C12N 15/12, C12N 15/62

(54) **PEPTIDES THERAPEUTIQUES ET LEUR UTILISATION CONTRE LA CHOREE DE HUNTINGTON**
THERAPEUTISCHE PEPTIDE UND IHRE VERWENDUNG FÜR MORBUS HUNTINGTON.
THERAPEUTIC PEPTIDES AND THEIR USE FOR HUNTINGTON'S DISEASE.

(30) Priorité: 12.04.2011 FR 1153193
(43) Date de publication de la demande: 19.02.2014
(73) Titulaire: Centre National de la Recherche Scientifique (C.N.R.S.), 75794 Paris Cedex 16 (FR); Institut National de la Santé et de la Recherche Médicale (INSERM), 75013 Paris (FR); Université de Montpellier, 34090 Montpellier (FR)
(72) Inventeur: MASCHAT, Florence, 34160 Boisseron (FR); PARMENTIER, Marie-Laure, 34000 Montpellier (FR); BONNEAUD, Nathalie, 34070 Montpellier (FR); ARRIBAT, Yoan, 34090 Montpellier (FR)
(74) Mandataire: Monni, Richard
(86) Numéro de dépôt international: PCT/FR2012/050809
(87) Numéro de publication internationale: WO 2012/140376

(56) Documents cités:
- WO-A2-01/68678
- WO-A2-2006/078648
- POPIEL H AKIKO ET AL: "Protein transduction domain-mediated delivery of QBP1 suppresses polyglutamine-induced neurodegeneration in vivo.", MOLECULAR THERAPY : THE JOURNAL OF THE AMERICAN SOCIETY OF GENE THERAPY FEB 2007 LNKD- PUBMED:17235308, vol. 15, no. 2, février 2007 (2007-02), pages 303-309, XP002669683, ISSN: 1525-0016
- WADA S ET AL: "Rationale for Antiangiogenic Cancer Therapy with Vaccination Using Epitope Peptides Derived from Human Vascular Endothelial Growth Factor Receptor 2", CANCER RESEARCH, AMERICAN ASSOCIATION FOR CANCER RESEARCH, US, vol. 65, no. 11, 1 juin 2005 (2005-06-01), pages 4939-4946, XP002348368, ISSN: 0008-5472, DOI: 10.1158/0008-5472.CAN-04-3759
- MUGAT BRUNO ET AL: "Protective role of Engrailed in a Drosophila model of Huntington's disease", HUMAN MOLECULAR GENETICS, vol. 17, no. 22, 20 août 2008 (2008-08-20) , pages 3601-3616, XP002669684, cité dans la demande
- DATABASE Geneseq [Online] 29 avril 2010 (2010-04-29), "Huntingtin proteolysis fragment protein sequence, SEQ ID NO: 1.", XP002669682, extrait de EBI accession no. GSP:AXW36976 Database accession no. AXW36976 & WO 2010/017408 A1 (BUCK INST FOR AGE RES [US]; UNIV CALIFORNIA [US]; ELLERBY LISA M [US];) 11 février 2010 (2010-02-11)
- NAGAI Y ET AL: "INHIBITION OF POLYGLUTAMINE PROTEIN AGGREGATION AND CELL DEATH BY NOVEL PEPTIDES IDENTIFIED BY PHAGE DISPLAY SCREENING", JOURNAL OF BIOLOGICAL CHEMISTRY, THE AMERICAN SOCIETY OF BIOLOGICAL CHEMISTS, INC, US, vol. 275, no. 14, 7 avril 2000 (2000-04-07), pages 10437-10442, XP002938480, ISSN: 0021-9258, DOI: 10.1074/JBC.275.14.10437

## Description

La présente invention concerne de nouveaux composés thérapeutiques contre la chorée de Huntington. Ces composés thérapeutiques comportent, ou codent pour, une séquence peptidique particulière.

La chorée de Huntington (HD) est une maladie rare neurodégénérative génétique, qui affecte à la fois les fonctions motrices et cognitives, conduisant la personne atteinte à un état de démence. De plus, bien que la maladie ne soit pas en elle-même fatale, elle induit des complications de santé (pneumonies, maladies cardiaques) réduisant l'espérance de vie de la personne atteinte à une vingtaine d'années suivant l'apparition des symptômes.

Il a été démontré que cette maladie est causée par une unique mutation génétique du gène codant pour la protéine Huntingtin humaine (hHtt). Il en résulte une synthèse d'une protéine anormale, qui a pour effet d'induire des désordres neuronaux dans le cerveau de la personne atteinte de la maladie.

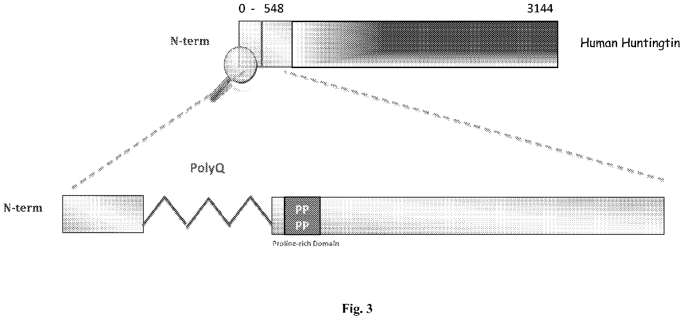

La figure 3 est un schéma du gène de la forme humaine de la Huntingtin (hHtt). Ce gène comporte 3144 acides aminés. La partie N-terminale (N-term) de ce gène est plus particulièrement détaillée. Cette partie N-term comporte 548 acides aminés (dont un domaine riche en acides aminés proline) plus un domaine polyglutamique appelé PolyQ, de taille variable, allant de 0Q à 35Q pour une protéine normale. Ce domaine polyQ correspond à une séquence composée exclusivement d'acides aminés glutamine (dont l'abréviation à une lettre correspondante est « Q »).

Plus particulièrement, dans la chorée de Huntington, la protéine anormale se caractérise par une expansion anormale du domaine polyQ contenu dans la partie N-terminale de la protéine hHtt.

Quand ce domaine excède 35Q, la protéine polyQ-hHtt forme des agrégats et une dégénérescence des neurones du striatum (structure nerveuse impliquée dans la motricité) est observée. Plusieurs études ont également identifié une influence de la fonction normale de la protéine Huntingtin dans la maladie.

Or, il n'existe pas à ce jour de traitement efficace contre cette maladie.

Une des approches de traitement actuellement envisagées consiste à lutter contre l'agrégation des domaines polyQ de la protéine, à l'origine de la maladie.

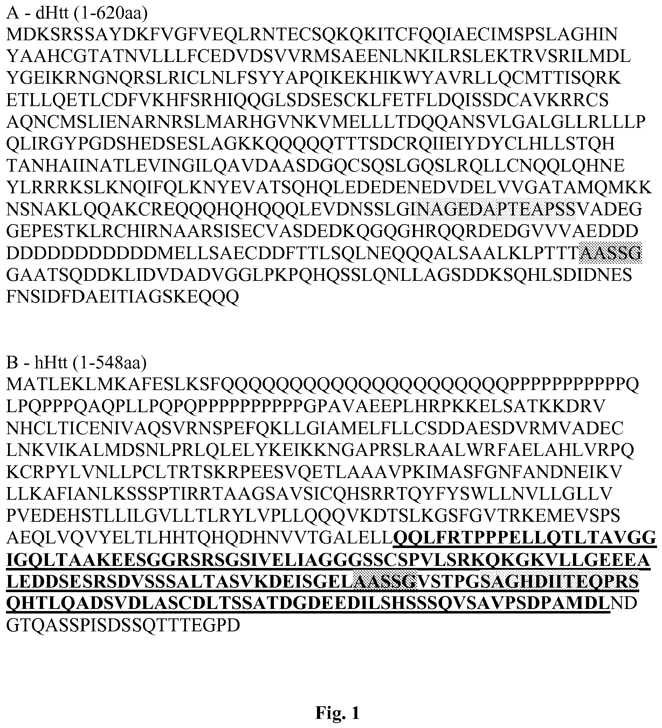

Une étude précédente (Mugat et al., Human Molecular Genetics, 2008) a permis de démontrer un effet protecteur des protéines sauvages hHtt et de son homologue drosophile dHtt concernant l'agrégation des protéines polyQ-hHtt. En effet, il a été possible de sauver les phénotypes induits par la protéine mutante polyQ-hHtt grâce à la présence de 548 acides aminés (aa) de la partie N-terminale (N-ter) de la protéine hHtt ou de 620aa de N-ter de l'homologue drosophile de l'Htt (dHtt). Ces séquences sont présentées en figure 1B et 1A respectivement. Sur la figure 1B, on note en première ligne, la présence de la série d'acides aminés glutamine (21Q au total) de cette protéine normale hHtt.

Toutefois, l'utilisation d'une protéine de 548 et/ou 620 acides aminés ne serait pas raisonnablement envisageable dans le cadre d'une thérapie génique. En effet, au vu des fonctions multiples exercées par la protéine huntingtin et en particulier par son extrémité N-terminale, ces protéines seraient également susceptibles d'exercer d'autres fonctions dont la nature n'est pas maîtrisée, [page 2bis]

L'invention concerne donc de nouveaux composés, particulièrement ciblés, utilisables comme médicament pour traiter la chorée de Huntington.

La présente divulgation concerne un peptide isolé présentant une taille inférieure ou égale à 200 acides aminés, de préférence 100 et comportant :
- une première séquence présentant au moins 80% d'identité avec la séquence AASSG (SEQ ID N°1), et/ou
- une seconde séquence présentant au moins 80% d'identité avec la séquence XAGXDXXTEXPXS (SEQ ID N°2), dans laquelle X désigne un acide aminé quelconque.

Par « pourcentage d'identité », on vise le pourcentage de résidus identiques entre deux séquences.

De préférence, les première et seconde séquences du peptide selon l'invention présentent au moins 90% d'identité, plus préférentiellement au moins 95%.

Ces séquences ont été isolées parmi les parties N-ter de 548 et 620aa des protéines hHtt et dHtt, ayant un effet protecteur sur l'agrégation des protéines polyQ-hHtt.

Avantageusement, le peptide isolé comportera à la fois la première séquence et la seconde séquence.

200 acides aminés est une taille acceptable en thérapie par les peptides. Toutefois, une taille inférieure sera préférée afin d'optimiser la sélectivité du peptide. Avantageusement, le peptide présentera une taille inférieure ou égale à 100 acides aminés, de préférence inférieure ou égale à 80, encore plus préférentiellement inférieure ou égale à 50, et d'une façon encore préférentielle égale ou inférieure à 40, 39, 38, 37 ou 36.

Les peptides sont particulièrement avantageux pour une utilisation dans le cadre d'une thérapie par les peptides car ils peuvent être à la fois très efficaces et sélectifs, si très spécifiques. De plus, ils sont peu toxiques.

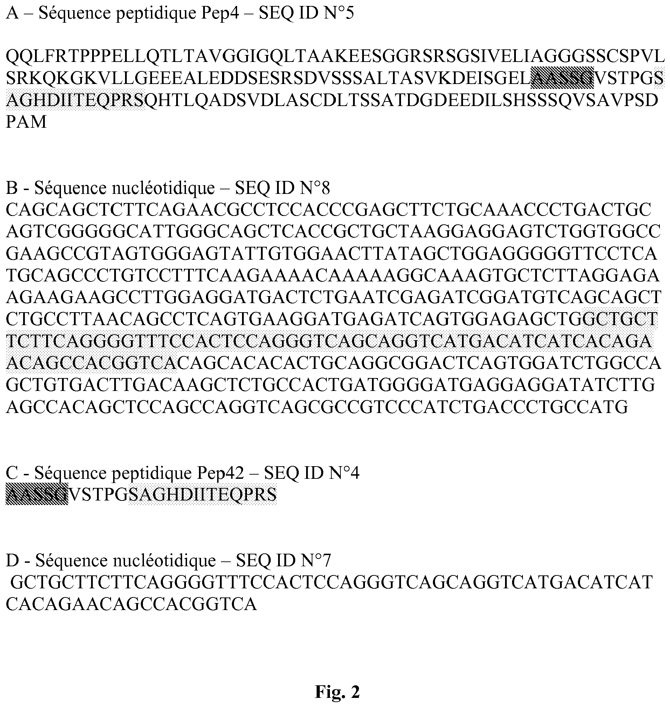

Selon un premier mode de réalisation, la seconde séquence présente la La recherche de peptides plus petits serait donc nécessaire. Une publication a mis en évidence un peptide de liaison aux polyQ (QBP1) issu d'une recherche combinatoire de peptides qui peut inhiber l'agrégation du polyQ (Popiel et al. Molecular Therapy : The Journal Of American Society of Gene Therapy, vol.15, no.2, 2007, pages 303-309) séquence suivante : SAGHDIITEQPRS (SEQ ID N°3). Plus particulièrement, le peptide comporte une séquence présentant au moins 80% d'identité avec la séquence AASSGVSTPGSAGHDIITEQPRS (SEQ ID N°4) ou la séquence QQLFRTPPPELLQTLTAVGGIGQLTAAKEESGGRSRSGSIVELIAGGGS SCSPVLSRKQKGKVLLGEEEALEDDSESRSDVSSSALTASVKDEISGELAASSGV STPGSAGHDIITEQPRSQHTLQADSVDLASCDLTSSATDGDEEDILSHSSSQVSAV PSDPAM (SEQ ID N°5). Ces séquences ont été appelées respectivement pep42 et pep4, comme indiqué en figure 2. Sur cette figure, on notera que pep4 et pep42 incluent les première et seconde séquences (indiqué par un fond opaque).

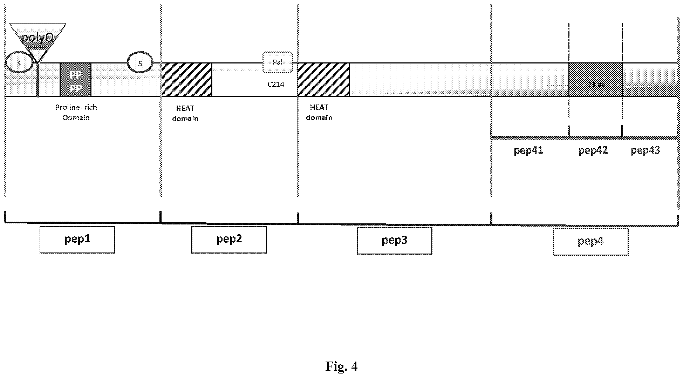

Pep4 puis pep42 ont été isolées à partir de la séquence de 548 aa incluant la partie N-ter de la protéine hHtt sauvage. En effet, comme indiqué en figure 4, la séquence de 548 aa incluant la partie N-ter de la protéine hHtt sauvage a été découpée en 4 sous-séquences désignées respectivement pep1, pep2, pep3 et pep4. De façon inattendue, c'est la sous-séquence pep4 qui s'est avérée être active (voir exemple 1). La sous-séquence pep4 a donc ensuite été elle-même divisée en 3 sous-fragments désignés pep41, pep42 et pep43, permettant la mise en évidence de l'activité d'une séquence de 23 aa, pep42 (voir exemple 2).

Pep4 et pep42 se sont avérés être capables de sauver la formation d'agrégats dans des cellules humaines, mais aussi dans un organisme entier dans les cellules non neuronales (telles que les glandes salivaires de la mouche) ou dans les motoneurones larvaires. Dans les exemples qui suivent, il a également été démontré que pep42 est capable de sauver les comportements physiologiques affectés par le polyQ-hHtt, tels que le trafic axonal des vésicules, la locomotion des larves ou encore la viabilité et longévité des mouches adultes. Enfin, il a pu être démontré que ce peptide, qui est localisé dans une région riche en sites de protéolyse, interagit avec l'extrémité N-terminale de l'Htt, empêchant ainsi le phénomène d'agrégation du polyQ-hHtt. [page 3bis]

Il a récemment été plus précisément identifié que Pep42 interagissait avec N17 (les 17 premiers aa) qui sont impliqués dans la première étape d'agrégation, appelée nucléation. Ceci suggère que Pep42 cible directement la protéine polyQ-hHtt, ce qui aura un effet sur l'agrégation et sur les phénotypes qui en découlent. Ces données mettent en avant le potentiel thérapeutique de Pep42 qui combine: i) une action directe sur la protéine polyQ-hHtt, donc en amont de toute autre action délétère ; ii) une grande spécificité puisque les séquences impliquées sont des régions spécifiques de l'Htt ; iii) peu de toxicité puisque l'action de Pep42 implique des domaines endogènes qui sont normalement présents dans l'organisme ; iV) les avantages des technologies peptidiques à des fins thérapeutiques.

Aussi, l'invention concerne un peptide isolé consistant en la séquence SEQ ID NO : 5, ou un fragment de celui-ci présentant une taille inférieure à 100 acides aminés et comprenant ou consistant en la séquence SEQ ID NO: 4, ou tout peptide ou fragment présentant au moins 80% d'identité avec la séquence SEQ ID NO : 4, et ayant un effet protecteur sur l'agrégation des protéines polyQ-hHtt.

Par « sauver », on entend donc que les peptides selon l'invention sont capables de faire disparaître les symptômes et/ ou les effets nocifs associés à la maladie en préservant et/ou restaurant les fonctions physiologiques normales.

De préférence, les séquences du peptide selon l'invention présentent au moins 90% d'identité, plus préférentiellement au moins 95% d'identité.

La seconde séquence présente la séquence suivante : NAGEDAPTEAPSS (SEQ ID N°6). Cette séquence correspond à la séquence identifiée dans la partie N-ter de 620aa de la protéine sauvage dHtt.

Quel que soit le mode de réalisation mis en oeuvre, le peptide selon l'invention sera avantageusement incorporé à une protéine de fusion, afin d'améliorer la pénétration du peptide selon l'invention dans le cytoplasme d'une cellule. L'invention vise donc également une protéine de fusion comportant un peptide selon l'invention et un domaine de transduction d'une protéine (PTD, « Protein Transduction Domain »). De préférence, le PTD est choisi parmi le groupe constitué par le peptide TAT « trans-acting activator of transcription » (SEQ ID N°11 : YGRKKRRQRRR), le peptide Penetratin™-1 (peptide de 16 acides aminés (SEQ ID N°12: RQIKIWFQNRRMKWKK) correspondant à la troisième hélice de l'homéodomaine de la protéine Antennapedia) et ses dérivés et le peptide actif de l'homéodomaine de la protéine Engrailed. Le peptide selon l'invention consiste notamment en la séquence SEQ ID NO : 13.

Lors de la synthèse du peptide fusion Pep42-TAT (SEQ ID N°13: AASSGVSTPGSAGHDIITEQPRSGGYGRKKRRQRRR), selon les techniques habituelles, il a été inséré 2 acide aminés (Glycine) entre la séquence Pep 42 et la séquence TAT, afin d'obtenir une plus grande flexibilité entre les deux domaines. Ce peptide fait également partie de l'invention.

Les peptides sont synthétisés en phase solide, en stratégie Fmoc. Ils sont ensuites analysés par spectrométrie de masse (ESI) afin de déterminer avec exactitude la masse moléculaire du peptide synthétisé. La comparaison entre la masse théorique et la masse expérimentale permet de confirmer l'absence de peptide de délétion ou de double couplage. La pureté des peptides synthétisés est déterminée par HPLC. Les peptides ainsi contrôlés et validés sont ensuite lyophilisés. Par ailleurs, du fait de la sensibilité des peptides et des protéines aux enzymes protéolytiques, ceux-ci sont facilement dégradés. Il est donc fortement recommandé de modifier chimiquement les extrémités des peptides et protéines selon l'invention afin de les protéger. De telles modifications chimiques peuvent être choisies parmi les acétylations et amidations.

De même, il est possible de stabiliser les peptides et les protéines par l'insertion d'acides aminés non naturels, tel que par exemple l'acide aminohexanoic. Dans le cas, d'une protéine de fusion avec TAT, il est possible d'insérer ces acides aminés non naturels dans la région riche en arginine de TAT.

Selon un second aspect de l'invention, l'invention concerne un polynucléotide codant pour un peptide ou une protéine décrit(e) ci-avant. En particulier, le polynucléotide est choisi parmi les polynucléotides comportant une séquence présentant au moins 80% d'identité avec les séquences suivantes :

Les séquences nucléotidiques SEQ ID N°7 et SEQ ID N°8 codent respectivement pour les peptides de séquences SEQ ID N°4 et SEQ ID N°5.

De préférence, les séquences des polynucléotides selon l'invention présentent au moins 90% d'identité, plus préférentiellement au moins 95% avec les séquences SEQ ID N°7 ou SEQ ID N°8.

L'invention vise également un vecteur d'expression comportant un polynucléotide selon l'invention. Avantageusement, le vecteur est de type virus, préférentiellement, de type lentivirus. En effet, les vecteurs de type lentivirus permettent de transfecter nombreux types cellulaires et de favoriser de manière remarquable l'internalisation dans la cellule et la délivrance du polynucléotide. Dans certains cas, ils permettent d'obtenir également une incorporation durable du polynucléotide dans le génome de la cellule hôte.

L'invention couvre également une cellule hôte comportant un vecteur d'expression selon l'invention.

Les peptides selon l'invention permettent donc d'empêcher la formation d'agrégats induits par les protéines anormales PolyQ-hHtt de la chorée de Huntington.

Les peptides, les protéines, les polynucléotides et les vecteurs selon l'invention sont donc avantageusement utilisables pour la fabrication d'un médicament, en particulier d'un médicament pour le traitement de la chorée de Huntington.

L'invention concerne également une composition pharmaceutique comportant une quantité efficace d'un peptide, d'une protéine, d'un polynucléotide ou d'un vecteur selon l'invention, avec un véhicule pharmaceutiquement acceptable.

Avantageusement, la composition pharmaceutique selon l'invention comporte en outre un second composé choisi parmi le groupe constitué par les peptides dirigés contre le polyQ, tels que QBP1 (pour « Polyglutamine-Binding Peptide 1 »), et les composés actifs contre la chorée de Huntington. Parmi les composés actifs contre la chorée de Huntington, on peut citer les anticorps simple chaîne (« intrabodies ») dirigés contre la chorée de Huntington.

Il est également divulgué une méthode de traitement thérapeutique comportant l'administration d'une quantité efficace d'un peptide, d'une protéine, d'un polynucléotide ou d'un vecteur selon l'invention à un patient atteint de la chorée de Huntington.

L'invention sera mieux comprise à la lecture de la description qui va suivre, donnée uniquement à titre d'exemple et faite en se référant aux dessins dans lesquels :
- la figure 1 présente les portions N-terminales des séquences des protéines Huntingtin de la drosophile (A) et humaine (B),
- la figure 2 présente les séquences des peptides Pep4 (A) et Pep42 (C) et leur séquence nucléotidique respective (B, D),
- la figure 3 est un schéma de la protéine Huntingtin humaine (hHtt) mettant en évidence la partie N-terminale,
- la figure 4 est un schéma du découpage d'une séquence de 548aa comportant la partie N-terminale présentée en figure 3, le découpage correspondant aux différentes sous-séquences étudiées,
- la figure 5 illustre l'effet protecteur du peptide Pep42, sur des cellules HeLa exprimant la protéine PolyQ-hHtt,
- la figure 6 illustre l'effet protecteur du peptide Pep42, sur le phénotype de dépigmentation et de dégénérescence des yeux de drosophiles exprimant le peptide polyQ-hHtt,
- la figure 7 illustre la diffusion et l'activité du peptide de fusion Pep42-TAT dans une culture de cellules HeLa, et,
- la figure 8 illustre la diffusion du peptide de fusion Pep42-TAT dans le cerveau de souris et sa stabilité à 24 heures.

### Exemple 1 : Identification de peptides inhibiteurs de l'agrégation de polyQ-hHtt

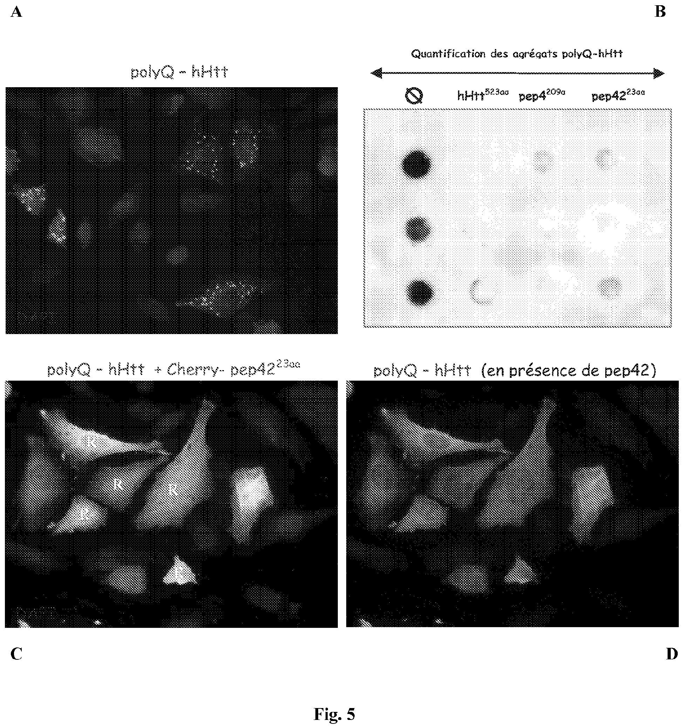

Différents peptides contenus dans les 548aa de la partie N-terminale de l'Huntingtin humaine ont été clonés par Gateway dans des vecteurs d'expression dans des cellules HeLa en culture, de type pcDNA. Chaque peptide a été taggué soit par Myc, soit par Cherry à son extrémité N-terminale. Ces différents peptides ont été testés par cotransfection dans des cellules HeLa en présence d'un vecteur pcDNA exprimant une protéine GFP-polyQ-hHtt (voir figure 5). Dans ces expériences, la protéine polyQ-hHtt testée correspondait à une protéine hHtt couvrant 171 aa et contenant un polyQ étendu avec un tag GFP à son extrémité N-ter (GFP- hHtt^{171aa}- 136Q). Les cellules HeLa ont été mises en culture dans des boites à 6 puits et transfectées par 1,5µg d'ADN total en utilisant du réactif JetPei (Qbiogene). Des quantités équivalentes de vecteurs d'expression (protéine polyQ et peptides) sont utilisées pour les cotransfections. Quand nécessaire, du pBluescript est utilisé pour équilibrer la quantité d'ADN. L'agrégation du GFP-hHtt^{171aa}-136Q est visualisée par immunodétection avec un anticorps anti-GFP (voir figure 5). Cette protéine forme des agrégats cytoplasmiques (voir figure 5A). En présence de pep42, détecté en rouge sur la figure 5C (symbolisé par la présence de la lettre R), la protéine GFP- hHtt^{171aa}-136Q ne forme plus d'agrégats comme cela est visible sur les figures 5C et 5D en comparaison avec la figure 5A. Cette analyse a permis d'identifier un effet inhibiteur de l'agrégation du polyQ-hHtt (couvrant 166aa entre les acides aminés 382 et 548, voir figure 2A) par pep4 et par le peptide pep42 de 23aa contenu dans pep4. Ce résultat est confirmé par quantification comme cela est montré dans l'exemple 3 qui suit.

### Exemple 2 : Propriétés inhibitrices de pep42 sur l'agrégation de polyQ-hHtt chez la drosophile

Les peptides pep4 et pep42 identifiés comme inhibiteurs de l'agrégation du polyQ-hHtt dans les cellules HeLa ont été clonés par la technique Gateway dans des vecteurs d'expression pUASt de drosophile, permettant l'expression de peptides taggués (par 6 Tag Myc ou GFP) à leur extrémité N-terminale.

Des mouches transgéniques exprimant ces peptides ont été construites par injection des vecteurs dans des embryons de drosophile. Le système UAS/Gal4 (Brand et Perrimon, Development. 1993) a été utilisé afin d'exprimer ces peptides à des temps et dans des tissus spécifiques, en présence d'un vecteur UAS-polyQ-hHtt, taggué HA (hémagglutinine).

Le pilote MS1096-Gal4 a été utilisé pour exprimer la protéine HA-hHtt^{171aa}-136Q (détecté par un anticorps anti-HA) dans les glandes salivaires, en absence ou en présence du peptide GFP-pep42. En présence du peptide GFP-pep42, détecté par un anti-GFP, la protéine polyQ-hHtt perd la capacité de s'agréger. Les immunomarquages ont été effectués sur des glandes salivaires de larves de 3ème stade disséquées. Les glandes sont fixées pendant 20 minutes dans du tampon PBS en présence de 3,7% de paraformaldéhyde (PFA) et Triton 0.1%, puis lavées en PBS/ Triton 0,1%. Les incubations avec les anticorps (anti-HA, puis anticorps conjugué au Cy3) sont effectuées en présence de 1% BSA. Ces immunomarquages ont montré l'absence d'agrégats en présence de pep4 ou de pep42.

### Exemple 3 : Quantification des agrégats par filtration

### a) A partir de cellules HeLa.

Des extraits protéiques ont été obtenus selon (Sittler et al., Mol Cell. 1998) avec les modifications suivantes : après transfection, les culots de cellules sont traités à la DNase et resuspendus dans 150µl 1% SDS et 50mM DTT dans du PBS. Les échantillons sont mis à bouillir 5min. Deux aliquots de 150µl correspondants à des expériences indépendantes ont été filtrés ensemble et chaque point a été reproduit 3 fois.

### b) A partir des glandes salivaires.

Les glandes de 3 larves de 3ème stade ont été disséquées et écrasées dans 30µl de 2% SDS et 50mM DTT, puis dénaturées 7min à 98°C. Les échantillons sont ensuite dilués dans 200µl de SDS 0,1% avant filtration. Pour chaque génotype, les échantillons ont été dupliqués.

Les échantillons ont été filtrés sur une membrane d'acétate de cellulose (0,2µM, Schleicher et Schuell) avec un appareil Dot-blot de Biorad. Les membranes ont ensuite été soumises à une immunodétection avec un anticorps soit polyclonal anti-GFP pour les cellules HeLa (Invitrogen, 1 : 5000), soit polyclonal anti-HA pour les glandes salivaires (SC805, Santa-Cruz, 1 : 200), permettant de visualiser les agrégats. Les agrégats sont ensuite détectés par un anticorps secondaire anti-lapin couplé à l'HRP (« horseradish peroxidase », Jackson 1 : 50 000). Les spots immunoréactifs sont détectés avec un substrat électrochimioluminescent (ECL, Roche) et les quantifications effectuées par Image J (voir figure 5B).

### Exemple 4 : Propriétés inhibitrices de pep42 sur la toxicité de l'oeil

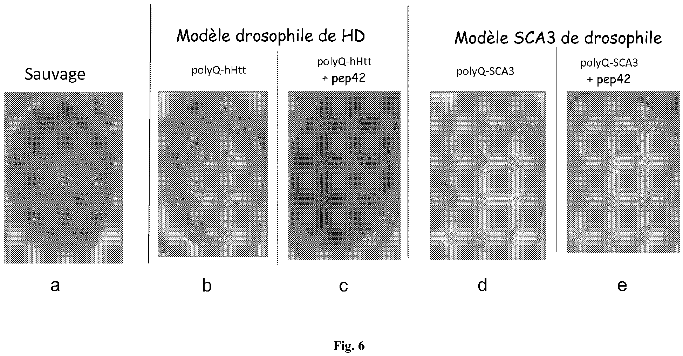

Les phénotypes de dépigmentation et de dégénérescence des yeux sont analysés chez des femelles âgées de 10 jours exprimant soit UAS-hHtt^{67aa}-98Q, soit UAS-hHtt^{548aa}-128Q sous contrôle du pilote gmr-Gal4, après un transfert à 29°C dès l'embryogénèse (voir la figure 6b, à comparer avec les mouches sauvages présentées en figure 6a). Des mouches exprimant d'autres gènes impliqués dans des maladies à polyQ (UAS-SCA1-polyQ ou UAS-SCA3-polyQ) ont également été analysées (figure 6d). Dans tous les cas, les mouches présentent une forte dégénérescence des yeux en absence du peptide pep42 comme cela est illustré en figures 6b et 6d. Seules les mouches exprimant les polyQ-hHtt (67aa ou 548aa) ont des yeux normaux en présence de pep42 figure 6c à comparer avec la figure 6e). Ces résultats montrent la spécificité de l'action de pep42 sur l'Huntingtin.

### Exemple 5 : Propriétés inhibitrices de l'Huntingtin normale et de pep42 sur le transport axonal affecté par l'expression de polyQ-hHtt

Afin de tester l'impact du polyQ-hHtt sur le transport axonal, le devenir de vésicules exprimant le Neuropeptide Y (NPY) marqué à la GFP (NPY-GFP) a été suivi, soit après dissection et fixation des larves, soit en microscopie en temps réel afin de suivre le trafic des vésicules dans les motoneurones.

### a) Fixation des larves.

Le système nerveux de larves OK6-Gal4 qui expriment des vésicules contenant le NPY-GFP (OK6-Gal4 ; UAS-NPY-GFP) a été disséqué et fixé comme précédemment dans du 3,7% PFA. Les incubations avec les anticorps et lavages sont effectuées dans du PBS, Triton 0,3%. L'expression de l'Htt^{548aa}-0Q normale n'a pas d'effet sur les vésicules qui restent nombreuses et également réparties le long de l'axone. L'Htt normale, détectée par un anticorps anti-Htt HU-4C8 (Chemicon, 1 :750), se retrouve le long de l'axone et n'est pas détectable au niveau des jonctions neuromusculaires (JNMs). L'expression de la protéine hHtt^{5a8aa}-128Q conduit à la formation d'agrégats le long des axones et à une accumulation anormale de la protéine au niveau des JNMs. Il s'ensuit une diminution du nombre global de vésicules et leur accumulation, surtout au niveau des agrégats. Dans tous les cas, les vésicules atteignent les JNMs. L'addition de pep42 permet d'observer une diminution de la taille des agrégats polyQ-hHtt, sa non-accumulation au niveau des JNMs et une meilleure répartition des vésicules le long des axones.

### b) Microscopie en temps réel.

Dans ce but, des larves OK6-Gal4 ; UAS-NPY-GFP ont été anesthésiées à l'éther pendant 2min et maintenues avec la face ventrale vers le haut entre lame et lamelle dans de l'agarose 1% polymérisé, pour une visualisation directe en microscopie à fluorescence avec un objectif 63X, afin de suivre le mouvement des vésicules entre les segments A3 et A4. Un film de 100 prises est effectué toutes les 280ms. Pour chaque film, 20 vésicules ont été analysées par Image J (Plugin « manual vesicle tracking » développé par F. Cordelières, Institut Curie, Orsay, France). Sont considérés en pause, des vésicules dont la vitesse instantanée est inférieure à 0,01µm/s. La vitesse moyenne est calculée comme la distance totale effectuée quelle que soit la direction de la vésicule (antérograde ou rétrograde) sur le temps total estimé pendant lequel la vésicule est suivie. Alors que l'expression de la protéine hHtt^{548aa}-128Q conduit à une augmentation du temps de pause des vésicules et à une diminution de leur vitesse, la présence de pep42 sauve à la fois le temps de pause et la vitesse des vésicules.

### c) Locomotion des larves.

Associés à ces problèmes vésiculaires des larves OK6-Gal4 ; UAS-NPY-GFP ; UAS-hHtt^{548aa}-128Q, une mobilité 25% plus faible des larves a été notée, mobilité qui est complètement sauvée en présence du pep42.

### Exemple 6 : Mécanisme d'action de pep42

L'effet protecteur de pep42 peut s'expliquer soit via une interaction directe avec l'Huntingtin polyQ, soit parce que pep42 titre des facteurs impliqués avec l'htt polyQ dans la maladie. Il a donc été recherché si pep42 pouvait interagir avec l'Htt et avec quelle partie. Pep42 est localisé entre les positions 480 et 502 de la protéine sauvage et est capable de sauver des phénotypes induits par des protéines polyQ-hHtt exprimant différentes tailles du N-ter (67aa, 170aa ou 548aa). Ceci suggère que si pep42 agit via une interaction directe avec l'Htt, cette interaction doit se faire avec les 67 premiers aa.

Cette hypothèse a été testée par des expériences de co-immunoprécipitation à partir de cellules HeLa cotransfectées par GFP-hHtt^{171aa}- 136Q ou par GFP-P1 (correspondant aux 98 premiers aa sans domaine polyQ), en présence de Cherry-pep42. Une résine de protéine G/A-agarose liant un anti-GFP a permis d'immunoprécipiter la protéine GFP-hHtt^{171aa}-136Q ou le peptide GFP-P1 et de co-immuoprécipiter le Cherry-pep42, visualisé par un anti-cherry par western blot. Ces résultats montrent que pep42 interagit en effet directement avec la partie N-terminale de l'Huntingtin, et que cette interaction ne nécessite pas la présence du domaine polyQ, ce qui explique la spécificité de l'effet protecteur de pep42 sur la chorée de Huntington, mais pas des autres maladies à polyQ.

Les mêmes expériences ont été répétées avec succès en utilisant les 17 premiers acides aminés avant le domaine polyQ de la protéine Htt humaine.

### Exemple 7 : Propriété de diffusion du peptide de fusion TAT-Pep42 et conservation de l'activité protectrice

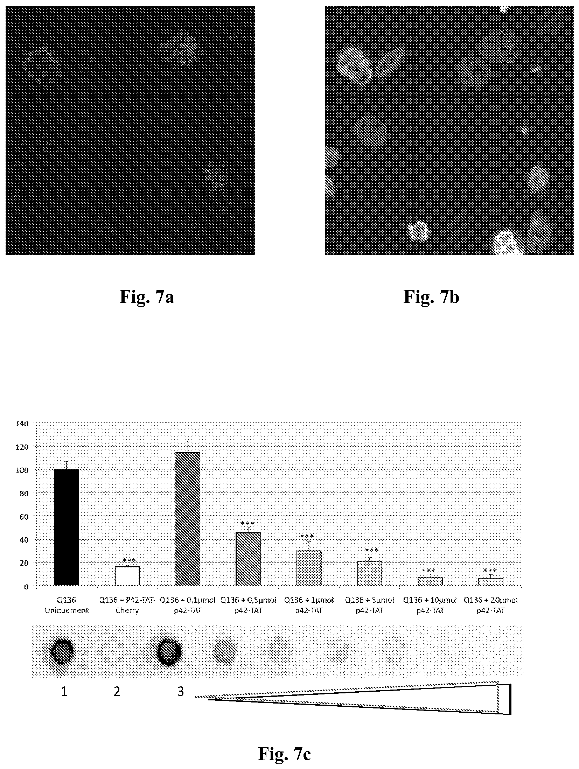

### • Diffusion dans des cellules HeLa (Figure 7a)

Le marqueur fluorescent TAMRA (Tetramethylrhodamine 5-Carboxamido-(6-Azidohexanyl) est couplé au pep42-TAT de manière à obtenir le peptide de fusion TAMRA-pep42-TAT. Du TAMRA-pep42-TAT est ajouté dans un milieu de culture de cellules HeLa. On visualise ensuite la fluorescence de ces peptides en microscopie par fluorescence (Figure 7a) que l'on superpose à la même vue en microscopie par contraste de phase (Figure 7b). La figure 7b montre nettement que la fluorescence est bien délimitée à l'intérieur des cellules et que le peptide de fusion (TAMRA-PEP42-TAT) a diffusé du milieu de culture pour pénétrer dans le cytoplasme des cellules.

### • Activité dans les cellules HeLa (Figure 7c)

Des cellules HeLa sont transfectées avec :
1- GFP-polyQ-hHtt (Q136),
2- GFP-polyQ-hHtt co-transfecté avec un vecteur exprimant Pep42-TAT, ou
3- GFP-polyQ-hHtt en présence de quantité croissante de peptide de synthèse Pep42-TAT mis dans le milieu de culture.

La figure 7c, correspond au pourcentage d'agrégat identifié par le signal GFP lié à la chaîne polyQ-hHtt, en présence de quantité croissante du peptide de fusion. On observe que la quantité de GFP des cellules diminue significativement.

Ces expériences montrent la conservation de l'activité protectrice du peptide de fusion.

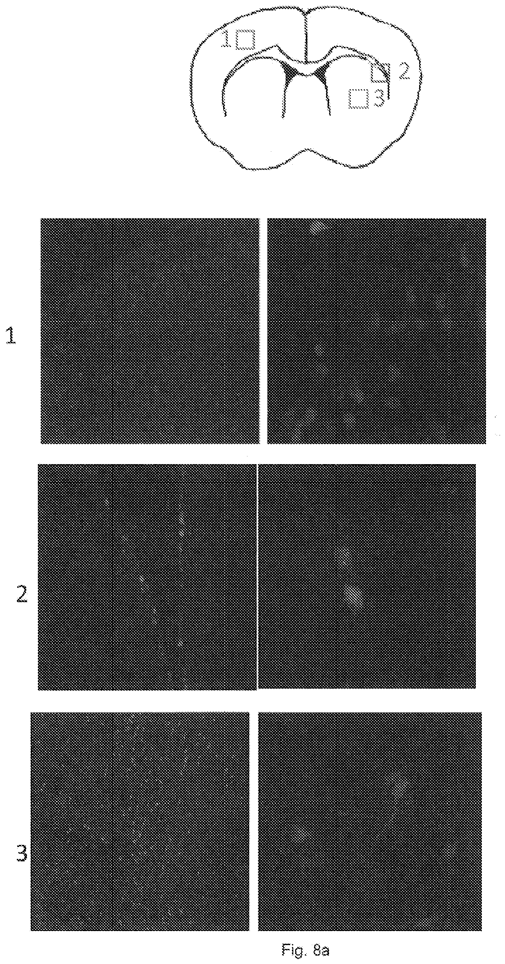

### • Diffusion dans le cerveau de souris (Figure 8a)

Une injection intracérébro-ventriculaire (ICV) de 5µg de TAMRA-Pep-42-TAT est effectuée au rythme de 1µg/min, suivie d'une analyse de coupes de cerveau soit 6 heures après l'injection (Figure 8a), soit 24 heure après l'injection.

Dans la figure 8a, on observe les imageries à deux grossissements distincts des différentes coupes suivantes:
1- Coupe du cortex gauche,
2- Coupe du ventricule droit, et
3- Coupe sous corticale (striatum).

Pour les trois coupes, on observe un marquage intra-cellulaire représenté par une coloration gris clair dans la figure.

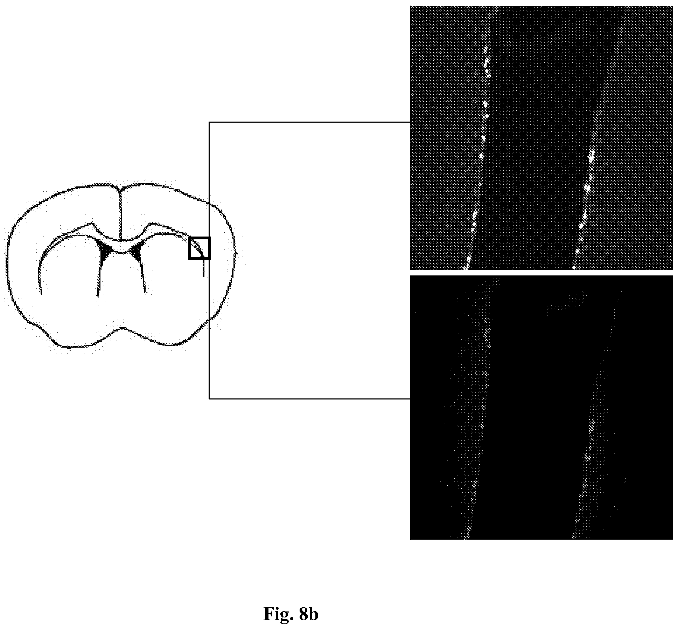

### • Stabilité à 24h (Figure 8b)

Une analyse de la fluorescence est effectuée au bout de 24 heure et on observe les résultats présentés en figure 8b qui démontre la présence du peptide de fusion près des ventricules et donc sa stabilité au bout de 24 heures.

## Revendications

1. Peptide isolé consistant en la séquence SEQ ID NO : 5, ou un fragment de celui-ci présentant une taille inférieure à 100 acides aminés et comprenant la séquence SEQ ID NO : 4, ou tout peptide ou fragment présentant au moins 80% d'identité avec la séquence SEQ ID NO : 4, et ayant un effet protecteur sur l'agrégation des protéines polyQ-hHtt.

2. Peptide isolé selon la revendication 1 consistant en la séquence SEQ ID N°4.

3. Protéine de fusion comportant un peptide selon la revendication 1 ou 2 et un domaine de transduction d'une protéine (PTD, « protein transduction domain »), ledit domaine de transduction étant notamment choisi parmi le groupe constitué par TAT (« trans-acting activator of transcription ») et le peptide Penetratin.

4. Protéine de fusion selon la revendication 3, ladite protéine de fusion consistant en la séquence SEQ ID NO : 13.

5. Polynucléotide codant un peptide selon la revendication 1 ou 2 ou codant une protéine selon la revendication 3 ou 4, notamment consistant en l'une quelconque des séquences SEQ ID NO : 7 et SEQ ID NO : 8.

6. Vecteur d'expression comportant un polynucléotide selon la revendication 5, ledit vecteur étant notamment de type virus.

7. Cellule hôte comportant un vecteur d'expression selon la revendication 6.

8. Composition pharmaceutique comportant une quantité efficace
- d'un peptide selon la revendication 1 ou 2, ou
- d'une protéine selon la revendication 3 ou 4, ou
- d'un polynucléotide selon la revendication 5, ou
- d'un vecteur selon la revendication 6,
en association avec un véhicule pharmaceutiquement acceptable, ladite composition comprenant notamment un second composé choisi parmi le groupe constitué par les peptides dirigés contre le polyQ et les composés actifs contre la chorée de Huntington.

9. Peptide isolé selon l'une quelconque des revendications 1 ou 2, ou protéine selon la revendication 3 ou 4,
pour une utilisation comme médicament.

10. Polynucléotide codant un peptide ou une protéine selon la revendication 9, pour son utilisation en tant que médicament.

11. Vecteur d'expression pour son utilisation en tant que médicament comportant un polynucléotide selon la revendication 10, ledit vecteur étant notamment de type virus.

12. Peptide selon l'une quelconque des revendications 1 ou 2, ou protéine selon la revendication 3 ou 4, ou polynucléotide selon la revendication 5, ou vecteur selon la revendication 6, pour son utilisation pour le traitement de la chorée de Huntington.

## Patentansprüche

1. Isoliertes Peptid, bestehend aus der Sequenz SEQ ID NO: 5, oder ein Fragment von diesem, das eine Größe kleiner als 100 Aminosäurem aufweist und die SEQ ID NO: 4 enthält, oder jedes beliebige Peptid oder Fragment, das mindestens 80 % Identität mit der SEQ ID NO: 4 aufweist, und einen Schutzeffekt auf die Verklumpung der polyQ-hHtt-Proteine hat.

2. Isoliertes Peptid nach Anspruch 1, bestehend aus der Sequenz SEQ ID NO: 4.

3. Fusionsprotein, aufweisend ein Peptid nach Anspruch 1 oder 2 und eine Proteintransduktionsdomäne (PTD, "protein transduction domain"), wobei die Transduktionsdomäne insbesondere ausgewählt ist aus der Gruppe bestehend aus TAT ("trans-acting activator of transcription") und dem Peptid Penetratin.

4. Fusionsprotein nach Anspruch 3, wobei das Fusionsprotein aus der Sequenz SEQ ID NO: 13 besteht.

5. Polynukleotid, das für ein Peptid nach Anspruch 1 oder 2 kodiert oder für ein Protein nach Anspruch 3 oder 4 kodiert, insbesondere bestehend aus einer der Sequenzen SEQ ID NO: 7 und SEQ ID NO: 8.

6. Expressionsvektor, der ein Polynukleotid nach Anspruch 5 aufweist, wobei der Vektor insbesondere des Virustyps ist.

7. Wirtszelle, die einen Expressionsvektor nach Anspruch 6 aufweist.

8. Pharmazeutische Zusammensetzung, aufweisend eine wirksame Menge
- eines Peptids nach Anspruch 1 oder 2, oder
- eines Proteins nach Anspruch 3 oder 4, oder
- eines Polynukleotids nach Anspruch 5, oder
- eines Vektors nach Anspruch 6
in Verbindung mit einem pharmazeutisch verträglichen Träger, wobei die Zusammensetzung insbesondere eine zweite Verbindung enthält, die ausgewählt ist aus der Gruppe, bestehend aus den Peptiden, die gegen PolyQ gerichtet sind, und den gegen die Chorea Huntington aktiven Verbindungen.

9. Isoliertes Peptid nach einem der Ansprüche 1 oder 2, oder Protein nach Anspruch 3 oder 4, für eine Verwendung als Arzneimittel.

10. Polynukleotid, das für ein Peptid oder ein Protein nach Anspruch 9 kodiert, für seine Verwendung als Arzneimittel.

11. Expressionsvektor für seine Verwendung als Arzneimittel, der ein Polynukleotid nach Anspruch 10 aufweist, wobei der Vektor insbesondere des Virustyps ist.

12. Peptid nach einem der Ansprüche 1 oder 2, oder Protein nach Anspruch 3 oder 4, oder Polynukleotid nach Anspruch 5, oder Vektor nach Anspruch 6, für seine Verwendung bei der Behandlung die Chorea Huntington.

## Claims

1. Isolated peptide consisting of the sequence SEQ ID NO: 5, or a fragment thereof, having a size smaller than 100 amino acids and comprising the sequence SEQ ID NO: 4, or any peptide or fragment having at least 80% identity with the sequence SEQ ID NO: 4, and having a protective effect on the aggregation of polyQ-hHtt proteins.

2. Isolated peptide according to Claim 1, consisting of the sequence SEQ ID NO: 4.

3. Fusion protein comprising a peptide according to Claim 1 or 2 and a protein transduction domain (PTD), said transduction domain being especially chosen from the group consisting of TAT (trans-acting activator of transcription) and the peptide penetratin.

4. Fusion protein according to Claim 3, said fusion protein consisting of the sequence SEQ ID NO: 13.

5. Polynucleotide coding for a peptide according to Claim 1 or 2 or coding for a protein according to Claim 3 or 4, especially consisting of either one of the sequences SEQ ID NO: 7 and SEQ ID NO: 8.

6. Expression vector comprising a polynucleotide according to Claim 5, said vector especially being of virus type.

7. Host cell comprising an expression vector according to Claim 6.

8. Pharmaceutical composition comprising an effective amount
- of a peptide according to Claim 1 or 2, or
- of a protein according to Claim 3 or 4, or
- of a polynucleotide according to Claim 5, or
- of a vector according to Claim 6,
in combination with a pharmaceutically acceptable carrier, said composition comprising especially a second compound chosen from the group consisting of peptides directed against polyQ and compounds active against Huntington's chorea.

9. Isolated peptide according to either one of Claims 1 and 2, or protein according to Claim 3 or 4, for its use as a medicament.

10. Polynucleotide coding for a peptide or a protein according to Claim 9, for its use as a medicament.

11. Expression vector, for its use as a medicament comprising a polynucleotide according to Claim 10, said vector especially being of virus type.

12. Peptide according to either one of Claims 1 and 2, or protein according to Claim 3 or 4, or polynucleotide according to Claim 5, or vector according to Claim 6, for its use for the treatment of Huntington's chorea.


### SEQUENCE LISTING

<110> CNRS, INSERM, UNIVERSITE MONTPELLIER 2, Centre National de la Recherche Scientifique et al
<120> Composés thérapeutiques et leur utilisation contre la chorée de Huntington
<130> FR 1153193
<140> Not known
   <141> 2012-04-12
<160> 13
<170> PatentIn version 3.5
<210> 1
   <211> 5
   <212> PRT
   <213> Artificial Sequence
<220>
   <223> extracted from human Huntingtin
<400> 1
   Ala Ala Ser Ser Gly
   1               5
<210> 2
   <211> 13
   <212> PRT
   <213> derived from human Huntingtin
<220>
   <221> misc_feature
   <222> (1)..(1)
   <223> Xaa can be any naturally occurring amino acid
<220>
   <221> misc_feature
   <222> (4)..(4)
   <223> Xaa can be any naturally occurring amino acid
<220>
   <221> misc_feature
   <222> (6)..(7)
   <223> Xaa can be any naturally occurring amino acid
<220>
   <221> misc_feature
   <222> (10)..(10)
   <223> Xaa can be any naturally occurring amino acid
<220>
   <221> misc_feature
   <222> (12)..(12)
   <223> Xaa can be any naturally occurring amino acid
<400> 2
   Xaa Ala Gly Xaa Asp Xaa Xaa Thr Glu Xaa Pro Xaa Ser
   1               5                   10
<210> 3
   <211> 13
   <212> PRT
   <213> Artificial Sequence
<220>
   <223> extracted from human Huntingtin
<400> 3
   Ser Ala Gly His Asp Ile Ile Thr Glu Gln Pro Arg Ser
   1               5                   10
<210> 4
   <211> 23
   <212> PRT
   <213> Pep42
<400> 4
   Ala Ala Ser Ser Gly Val Ser Thr Pro Gly Ser Ala Gly His Asp Ile
   1               5                   10                  15
   Ile Thr Glu Gln Pro Arg Ser
               20
<210> 5
   <211> 166
   <212> PRT
   <213> Pep4
<400> 5
   Gln Gln Leu Phe Arg Thr Pro Pro Pro Glu Leu Leu Gln Thr Leu Thr
   1               5                   10                  15
   Ala Val Gly Gly Ile Gly Gln Leu Thr Ala Ala Lys Glu Glu Ser Gly
               20                  25                  30
   Gly Arg Ser Arg Ser Gly Ser Ile Val Glu Leu Ile Ala Gly Gly Gly
           35                  40                  45
   Ser Ser Cys Ser Pro Val Leu Ser Arg Lys Gln Lys Gly Lys Val Leu
       50                  55                  60
   Leu Gly Glu Glu Glu Ala Leu Glu Asp Asp Ser Glu Ser Arg Ser Asp
   65                  70                  75                  80
   Val Ser Ser Ser Ala Leu Thr Ala Ser Val Lys Asp Glu Ile Ser Gly
                   85                  90                  95
   Glu Leu Ala Ala Ser Ser Gly Val Ser Thr Pro Gly Ser Ala Gly His
               100                 105                 110
   Asp Ile Ile Thr Glu Gln Pro Arg Ser Gln His Thr Leu Gln Ala Asp
           115                 120                 125
   Ser Val Asp Leu Ala Ser Cys Asp Leu Thr Ser Ser Ala Thr Asp Gly
       130                 135                 140
   Asp Glu Glu Asp Ile Leu Ser His Ser Ser Ser Gln Val Ser Ala Val
   145                 150                 155                 160
   Pro Ser Asp Pro Ala Met
                   165
<210> 6
   <211> 13
   <212> PRT
   <213> Drosophila
<400> 6
   Asn Ala Gly Glu Asp Ala Pro Thr Glu Ala Pro Ser Ser
   1               5                   10
<210> 7
   <211> 69
   <212> DNA
   <213> extracted from human Huntingtin
<400> 7
   gctgcttctt caggggtttc cactccaggg tcagcaggtc atgacatcat cacagaacag 60
   ccacggtca 69
<210> 8
   <211> 498
   <212> DNA
   <213> Artificial Sequence
<220>
   <223> extracted from human Huntingtin
<400> 8
   cagcagctct tcagaacgcc tccacccgag cttctgcaaa ccctgactgc agtcgggggc 60
   attgggcagc tcaccgctgc taaggaggag tctggtggcc gaagccgtag tgggagtatt 120
   gtggaactta tagctggagg gggttcctca tgcagccctg tcctttcaag aaaacaaaaa 180
   ggcaaagtgc tcttaggaga agaagaagcc ttggaggatg actctgaatc gagatcggat 240
   gtcagcagct ctgccttaac agcctcagtg aaggatgaga tcagtggaga gctggctgct 300
   tcttcagggg tttccactcc agggtcagca ggtcatgaca tcatcacaga acagccacgg 360
   tcacagcaca cactgcaggc ggactcagtg gatctggcca gctgtgactt gacaagctct 420
   gccactgatg gggatgagga ggatatcttg agccacagct ccagccaggt cagcgccgtc 480
   ccatctgacc ctgccatg 498
<210> 9
   <211> 620
   <212> PRT
   <213> Drosophila
<400> 9
   Met Asp Lys Ser Arg Ser Ser Ala Tyr Asp Lys Phe Val Gly Phe Val
   1               5                   10                  15
   Glu Gln Leu Arg Asn Thr Glu Cys Ser Gln Lys Gln Lys Ile Thr Cys
               20                  25                  30
   Phe Gln Gln Ile Ala Glu Cys Ile Met Ser Pro Ser Leu Ala Gly His
           35                  40                  45
   Ile Asn Tyr Ala Ala His Cys Gly Thr Ala Thr Asn Val Leu Leu Leu
       50                  55                  60
   Phe Cys Glu Asp Val Asp Ser Val Val Arg Met Ser Ala Glu Glu Asn
   65                  70                  75                  80
   Leu Asn Lys Ile Leu Arg Ser Leu Glu Lys Thr Arg Val Ser Arg Ile
                   85                  90                  95
   Leu Met Asp Leu Tyr Gly Glu Ile Lys Arg Asn Gly Asn Gln Arg Ser
               100                 105                 110
   Leu Arg Ile Cys Leu Asn Leu Phe Ser Tyr Tyr Ala Pro Gln Ile Lys
           115                 120                 125
   Glu Lys His Ile Lys Trp Tyr Ala Val Arg Leu Leu Gln Cys Met Thr
       130                 135                 140
   Thr Ile Ser Gln Arg Lys Glu Thr Leu Leu Gln Glu Thr Leu Cys Asp
   145                 150                 155                 160
   Phe Val Lys His Phe Ser Arg His Ile Gln Gln Gly Leu Ser Asp Ser
                   165                 170                 175
   Glu Ser Cys Lys Leu Phe Glu Thr Phe Leu Asp Gln Ile Ser Ser Asp
               180                 185                 190
   Cys Ala Val Lys Arg Arg Cys Ser Ala Gln Asn Cys Met Ser Leu Ile
           195                 200                 205
   Glu Asn Ala Arg Asn Arg Ser Leu Met Ala Arg His Gly Val Asn Lys
       210                 215                 220
   Val Met Glu Leu Leu Leu Thr Asp Gln Gln Ala Asn Ser Val Leu Gly
   225                 230                 235                 240
   Ala Leu Gly Leu Leu Arg Leu Leu Leu Pro Gln Leu Ile Arg Gly Tyr
                   245                 250                 255
   Pro Gly Asp Ser His Glu Asp Ser Glu Ser Leu Ala Gly Lys Lys Gln
               260                 265                 270
   Gln Gln Gln Gln Thr Thr Thr Ser Asp Cys Arg Gln Ile Ile Glu Ile
           275                 280                 285
   Tyr Asp Tyr Cys Leu His Leu Leu Ser Thr Gln His Thr Ala Asn His
       290                 295                 300
   Ala Ile Ile Asn Ala Thr Leu Glu Val Ile Asn Gly Ile Leu Gln Ala
   305                 310                 315                 320
   Val Asp Ala Ala Ser Asp Gly Gln Cys Ser Gln Ser Leu Gly Gln Ser
                   325                 330                 335
   Leu Arg Gln Leu Leu Cys Asn Gln Gln Leu Gln His Asn Glu Tyr Leu
               340                 345                 350
   Arg Arg Arg Lys Ser Leu Lys Asn Gln Ile Phe Gln Leu Lys Asn Tyr
           355                 360                 365
   Glu Val Ala Thr Ser Gln His Gln Leu Glu Asp Glu Asp Glu Asn Glu
       370                 375                 380
   Asp Val Asp Glu Leu Val Val Gly Ala Thr Ala Met Gln Met Lys Lys
   385                 390                 395                 400
   Asn Ser Asn Ala Lys Leu Gln Gln Ala Lys Cys Arg Glu Gln Gln Gln
                   405                 410                 415
   His Gln His Gln Gln Gln Leu Glu Val Asp Asn Ser Ser Leu Gly Ile
               420                 425                 430
   Asn Ala Gly Glu Asp Ala Pro Thr Glu Ala Pro Ser Ser Val Ala Asp
           435                 440                 445
   Glu Gly Gly Glu Pro Glu Ser Thr Lys Leu Arg Cys His Ile Arg Asn
   450 455 460
   Ala Ala Arg Ser Ile Ser Glu Cys Val Ala Ser Asp Glu Asp Lys Gln
   465 470 475 480
   Gly Gln Gly His Arg Gln Gln Arg Asp Glu Asp Gly Val Val Val Ala
   485 490 495
   Glu Asp Asp Asp Asp Asp Asp Asp Asp Asp Asp Asp Asp Asp Asp Met
   500 505 510
   Glu Leu Leu Ser Ala Glu Cys Asp Asp Phe Thr Thr Leu Ser Gln Leu
   515 520 525
   Asn Glu Gln Gln Gln Ala Leu Ser Ala Ala Leu Lys Leu Pro Thr Thr
   530 535 540
   Thr Ala Ala Ser Ser Gly Gly Ala Ala Thr Ser Gln Asp Asp Lys Leu
   545 550 555 560
   Ile Asp Val Asp Ala Asp Val Gly Gly Leu Pro Lys Pro Gln His Gln
   565 570 575
   Ser Ser Leu Gln Asn Leu Leu Ala Gly Ser Asp Asp Lys Ser Gln His
   580 585 590
   Leu Ser Asp Ile Asp Asn Glu Ser Phe Asn Ser Ile Asp Phe Asp Ala
   595 600 605
   Glu Ile Thr Ile Ala Gly Ser Lys Glu Gln Gln Gln
   610 615 620
<210> 10
   <211> 570
   <212> PRT
   <213> Human Huntingtin
<400> 10
   Met Ala Thr Leu Glu Lys Leu Met Lys Ala Phe Glu Ser Leu Lys Ser
   1 5 10 15
   Phe Gln Gln Gln Gln Gln Gln Gln Gln Gln Gln Gln Gln Gln Gln Gln
   20 25 30
   Gln Gln Gln Gln Gln Gln Pro Pro Pro Pro Pro Pro Pro Pro Pro Pro
   35 40 45
   Pro Gln Leu Pro Gln Pro Pro Pro Gln Ala Gln Pro Leu Leu Pro Gln
       50                  55                  60
   Pro Gln Pro Pro Pro Pro Pro Pro Pro Pro Pro Pro Gly Pro Ala Val
   65                  70                  75                  80
   Ala Glu Glu Pro Leu His Arg Pro Lys Lys Glu Leu Ser Ala Thr Lys
                   85                  90                  95
   Lys Asp Arg Val Asn His Cys Leu Thr Ile Cys Glu Asn Ile Val Ala
               100                 105                 110
   Gln Ser Val Arg Asn Ser Pro Glu Phe Gln Lys Leu Leu Gly Ile Ala
           115                 120                 125
   Met Glu Leu Phe Leu Leu Cys Ser Asp Asp Ala Glu Ser Asp Val Arg
       130                 135                 140
   Met Val Ala Asp Glu Cys Leu Asn Lys Val Ile Lys Ala Leu Met Asp
   145                 150                 155                 160
   Ser Asn Leu Pro Arg Leu Gln Leu Glu Leu Tyr Lys Glu Ile Lys Lys
                   165                 170                 175
   Asn Gly Ala Pro Arg Ser Leu Arg Ala Ala Leu Trp Arg Phe Ala Glu
               180                 185                 190
   Leu Ala His Leu Val Arg Pro Gln Lys Cys Arg Pro Tyr Leu Val Asn
           195                 200                 205
   Leu Leu Pro Cys Leu Thr Arg Thr Ser Lys Arg Pro Glu Glu Ser Val
       210                 215                 220
   Gln Glu Thr Leu Ala Ala Ala Val Pro Lys Ile Met Ala Ser Phe Gly
   225                 230                 235                 240
   Asn Phe Ala Asn Asp Asn Glu Ile Lys Val Leu Leu Lys Ala Phe Ile
                   245                 250                 255
   Ala Asn Leu Lys Ser Ser Ser Pro Thr Ile Arg Arg Thr Ala Ala Gly
               260                 265                 270
   Ser Ala Val Ser Ile Cys Gln His Ser Arg Arg Thr Gln Tyr Phe Tyr
           275                 280                 285
   Ser Trp Leu Leu Asn Val Leu Leu Gly Leu Leu Val Pro Val Glu Asp
   290 295 300
   Glu His Ser Thr Leu Leu Ile Leu Gly Val Leu Leu Thr Leu Arg Tyr
   305 310 315 320
   Leu Val Pro Leu Leu Gln Gln Gln Val Lys Asp Thr Ser Leu Lys Gly
   325 330 335
   Ser Phe Gly Val Thr Arg Lys Glu Met Glu Val Ser Pro Ser Ala Glu
   340 345 350
   Gln Leu Val Gln Val Tyr Glu Leu Thr Leu His His Thr Gln His Gln
   355 360 365
   Asp His Asn Val Val Thr Gly Ala Leu Glu Leu Leu Gln Gln Leu Phe
   370 375 380
   Arg Thr Pro Pro Pro Glu Leu Leu Gln Thr Leu Thr Ala Val Gly Gly
   385 390 395 400
   Ile Gly Gln Leu Thr Ala Ala Lys Glu Glu Ser Gly Gly Arg Ser Arg
   405 410 415
   Ser Gly Ser Ile Val Glu Leu Ile Ala Gly Gly Gly Ser Ser Cys Ser
   420 425 430
   Pro Val Leu Ser Arg Lys Gln Lys Gly Lys Val Leu Leu Gly Glu Glu
   435 440 445
   Glu Ala Leu Glu Asp Asp Ser Glu Ser Arg Ser Asp Val Ser Ser Ser
   450 455 460
   Ala Leu Thr Ala Ser Val Lys Asp Glu Ile Ser Gly Glu Leu Ala Ala
   465 470 475 480
   Ser Ser Gly Val Ser Thr Pro Gly Ser Ala Gly His Asp Ile Ile Thr
   485 490 495
   Glu Gln Pro Arg Ser Gln His Thr Leu Gln Ala Asp Ser Val Asp Leu
   500 505 510
   Ala Ser Cys Asp Leu Thr Ser Ser Ala Thr Asp Gly Asp Glu Glu Asp
   515 520 525
   Ile Leu Ser His Ser Ser Ser Gln Val Ser Ala Val Pro Ser Asp Pro
   530 535 540
   Ala Met Asp Leu Asn Asp Gly Thr Gln Ala Ser Ser Pro Ile Ser Asp
   545                 550                 555                 560
   Ser Ser Gln Thr Thr Thr Glu Gly Pro Asp
                   565                 570
<210> 11
   <211> 11
   <212> PRT
   <213> Artificial Sequence
<220>
   <223> Protein Transductor Domain
<400> 11
   Tyr Gly Arg Lys Lys Arg Arg Gln Arg Arg Arg
   1               5                   10
<210> 12
   <211> 16
   <212> PRT
   <213> extracted from antennapedia protein
<400> 12
   Arg Gln Ile Lys Ile Trp Phe Gln Asn Arg Arg Met Lys Trp Lys Lys
   1               5                   10                  15
<210> 13
   <211> 36
   <212> PRT
   <213> Pep42-TAT
<400> 13
   Ala Ala Ser Ser Gly Val Ser Thr Pro Gly Ser Ala Gly His Asp Ile
   1               5                   10                  15
   Ile Thr Glu Gln Pro Arg Ser Gly Gly Tyr Gly Arg Lys Lys Arg Arg
               20                  25                  30
   Gln Arg Arg Arg
           35